(19)

**Europäisches Patentamt**
**European Patent Office**
**Office européen des brevets**

(11) **EP 3 929 308 A1**

(12) **EUROPEAN PATENT APPLICATION**

(43) Date of publication:
**29.12.2021 Bulletin 2021/52**

(51) Int Cl.:
***C12Q 1/6883*** (2018.01)

(21) Application number: **20181630.3**

(22) Date of filing: **23.06.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Heinrich-Heine-Universität Düsseldorf 40225 Düsseldorf (DE)**

(72) Inventors:
• **Brandenburger, Timo**
  **40699 Erkrath (DE)**
• **DEVAUX, Yvan**
  **F-57330 Zoufftgen (FR)**

(74) Representative: **Gramm, Lins & Partner Patent- und Rechtsanwälte PartGmbB Freundallee 13a 30173 Hannover (DE)**

(54) **DIAGNOSTIC METHODS ON RENAL RECOVERY IN INDIVIDUALS SUFFERING FROM ACUTE KIDNEY INJURY AND SUITABLE BIOMARKERS THEREFOR**

(57) In a first aspect, the present invention relates to a method for determining renal recovery in individuals suffering from acute kidney injury (AKI). The method is based on determining the expression level or amount of at least one biomarker of miRNA and comparing the expression level or amount of the at least one miRNA with a reference value of said miRNA for allowing to determine whether the individual suffering from AKI is under or will undergo renal recovery. In addition, the use of a test kit or kit of parts for determining renal recovery in individuals suffering from AKI is provided based on the miRNA described herein. Finally, the *in vitro* use of at least one of the miRNA as defined herein is described.

EP 3 929 308 A1

SEQUENCE LISTING

<110>  Heinrich-Heine Universitaet Duesseldorf

<120>  Diagnostic methods on renal recovery in individuals suffering
       from acute kidney injury and suitable biomarkers therefor

<130>  4534-006 EP

<160>  8

<170>  PatentIn version 3.5

<210>  1
<211>  21
<212>  RNA
<213>  homo sapiens

<400>  1
cugaccuaug aauugacagc c                                                  21


<210>  2
<211>  22
<212>  RNA
<213>  homo sapiens

<400>  2
uguaacagca acuccaugug ga                                                 22


<210>  3
<211>  22
<212>  RNA
<213>  homo sapiens

<400>  3
ugggucuuug cgggcgagau ga                                                 22


<210>  4
<211>  23
<212>  RNA
<213>  homo sapiens

<400>  4
agcagcauug uacagggcua uca                                                23


<210>  5
<211>  22
<212>  RNA
<213>  homo sapiens

<400>  5
uggaguguga caaugguguu ug                                                 22


<210>  6
<211>  22
<212>  RNA
<213>  homo sapiens

```
<400>   6
ucccugagac ccuaacuugu ga                                          22


<210>   7
<211>   22
<212>   RNA
<213>   homo sapiens

<400>   7
uggcaguguc uuagcugguu gu                                          22


<210>   8
<211>   22
<212>   RNA
<213>   homo sapiens

<400>   8
cuccugacuc cagguccugu gu                                          22
```

**Description**

In a first aspect, the present invention relates to a method for determining renal recovery in individuals suffering from acute kidney injury (AKI). The method is based on determining the expression level or amount of at least one biomarker of miRNA and comparing the expression level or amount of the at least one miRNA with a reference value of said miRNA for allowing to determine whether the individual suffering from AKI is under or will undergo renal recovery. In addition, the use of a test kit or kit of parts for determining renal recovery in individuals suffering from AKI is provided based on the miRNA described herein. Finally, the *in vitro* use of at least one of the miRNAs as defined herein is described.

**Prior art**

Acute kidney injury (AKI) is a global problem affecting more than 10 % of all hospitalized patients and approaching up to two-thirds of those admitted to intensive care units. Survival rate is not only dependent on the severity of the disease but also on the duration of renal-dysfunction. Therefore, closely monitoring the renal dysfunction is required. It is speculated presently that two-thirds of patients with AKI resolve their renal dysfunction within 3 - 7 days whereas those who persist have dramatically reduced survival over the following year. AKI conveys significant morbidity and mortality, and is a major risk factor of chronic kidney disease and is thus associated with huge, health and social economic burdens. The link between AKI and chronic kidney disease has been established over the last decade and specific recommendations for the management of patients with AKI have been proposed in order to potentially influence this transition.

That is, early identification of individuals at risk of persistent AKI would enable appropriate delivery of these proposed interventions, but also may identify individuals where newer therapies to attenuate AKI could be targeted.

At present, renal replacement therapy or dialysis is the cornerstone of the management of severe acute kidney injury and the initial starting point of renal replacement therapy is of importance. At present many patients suffering from AKI undergo renal replacement therapy prophylactically, thus covering the 10 % of patients with severe degree on renal insufficiency requiring dialysis whereas about 90 % of the patients at day 0, namely, the day on establishing acute kidney injury and treating the individual on intensive care units, do not require dialysis, however, treatment is conducted prophylactically.

At present, it is suggested to delay initiation of renal replacement therapy unless certain parameter in the urine are identified which indicates that renal replacement therapy should be started. For example, Gaudry. S., et al., The New England Journal of Medicine 2016, 375, 122 - 133, suggest that a delayed-strategy averted the need for renal replacement therapy in an appreciable number of patients. Therein patients with severe hyperkalemia, metabolic acidosis, pulmonary edema, elevated blood urea nitrogen or an oliguria of at least 72h were treated with renal replacement therapy. Thus, the early identification of individuals at risk of persistent AKI would enable appropriate delivery of possible interventions. Moreover, from the literature discussion arose on the timing of renal replacement therapy with studies showing that some patients can benefit from the earlier initiation of a renal replacement therapy, while other studies demonstrates that some individuals receive renal replacement therapy who may not require such a treatment as they have an early recovery renal of function. Hence, early and reliable identification of those who will recover renal function may enable more satisfied approach to management, thus, avoiding the incumbent risk of futile extracorporal therapy.

In view of the present approach to start renal replacement therapy prophylactically and the risk associated therewith, it is desirable to allow to identify patients requiring renal replacement therapy and persons not requiring renal replacement therapy in the cohort of patients, the 90 % of patients in the intensive care unit which may not require renal replacement therapy when admitted to intensive care. That is, it will be of help to identify patients who may have renal function recovery soon, thus, not requiring renal replacement therapy.

Fan P-C., et al., Human Genomics, 2016, 10:29, describe microRNAs in acute kidney injury. It is mentioned therein, that in AKI some miRNAs appear to act pathogenically by promoting inflammation, apoptosis, and fibrosis, while others may act protectively by exerting anti-inflammatory, anti-apoptotic, anti-fibrotic, and pro-angiogenic effects. Hence, miRNAs have not only emerged as novel biomarkers for AKI, they also hold promise to be potential therapeutic targets. Moreover, EP 2 474 622 A1 refers to a method for the diagnosis and/or prognosis of acute renal damage. Therein methods for the diagnosis and/or prognosis of acute renal damage are described comprising analyzing a sample of a patient for determining the level of expression of microRNAs encomparing the same with a control value, thus, allowing to identify patients suffering from acute renal damage. Therein, analysis of samples obtained from urine have been described. However, a major issue on patients with AKI is oliguria or anuria, namely a low output of urine. Namely, it is well-known that patients suffering from AKI have problems to urinate. Consequently, it is difficult to identify marker molecules in urine from said patients accordingly. It is mentioned therein, that identifying miR-210 in the urine of patients may indicate the onset of recovery after renal ischemic damage.

Moreover, a recent study of Hoste E., et al., Intensive Care Medicine, 46, 943-953(2020) describes the RUBY study to evaluate novel-candidate biomarkers to enable prediction of persistence of renal dysfunction. It is identified therein that elevated urinary CCL 14 predicts persistent AKI in a large heterogenus cohort of critically ill patients with severe AKI while other markers failed.

That is, there is an ongoing need for methods and marker molecules allowing to determine renal recovery in individuals. This is a sharp contrast to markers which enable to identify persistent AKI in patients.

**Brief description of the present invention**

The aim of the present invention is to provide methods and markers allowing to determine renal recovery in individuals, thus, allowing at an early time point the differentiation of AKI patients having a high potential of renal recovery and those with little or no potential of recovery which may result in persistent AKI or death. That is, it is important to identify the patients undergoing spontaneous renal recovery after acute kidney injury. Patients with spontaneous renal recovery do not require treatment with renal replacement therapy and high costs of dialysis can be avoided. However, the patients with higher risk of renal insufficiency due to AKI can be treated by renal replacement therapy, hence, potentially avoiding AKI complications such as volume overload, electrolyte disorders and subsequently death.

The present inventors aim in providing a method based on specific biomarkers allowing to determine renal recovery in individuals suffering from AKI at an early time point.

In a first aspect, the present invention relates to a method for determining renal recovery in individuals suffering from acute kidney injury (AKI) comprising the steps of

a) providing a sample obtained from said individual suffering from AKI;
b) determining the expression level or amount of at least one of the miRNA selected from the group consisting of miR-192-5p, miR-194-5p, miR-107, miR-122-5p, miR-125b-5p, miR-193a-5p, miR-34a-5p, and miR-378a-3p;
c) comparing the expression level or amount of the at least one miRNA with a reference value of said miRNA;
d) determining whether the individual suffering from acute kidney injury is under renal recovery.

In particular, the expression level or amount of at least one of the miRNA miR-192-5p and miR-194-5p and at least one second miRNA whereby the at least one second miRNA i) is the other miRNA or miR-192-5p and miR-194-5p and/or ii) is selected from the group consisting of miR-107, miR-122-5p, miR-125b-5p, miR-193a-5p, miR-34a-5p, and miR-378a-3p.

In an embodiment, the sample obtained from the individual, is blood, like whole blood and, in particular, is blood plasma.

In an embodiment, the method according to the present invention for determining renal recovery in individuals, is conducted with a sample obtained from said individual within 72 hours, like 24 hours, e. g. within 12 hours after diagnosis of AKI.

In another embodiment, the present invention relates to the use of a test kit or kit of parts for determining renal recovery in individuals suffering from AKI. The kit or kit of parts comprising means for determining the expression level or amount of the miRNA to be determine according to the present invention and instructions of how to use said test kit or kit of parts in a method according to the present invention.

Further, the present invention relates to the *in vitro* use of at least one of the miRNA as defined herein in determining renal recovery in individuals suffering from AKI encomparing the expression level or amount with a reference value.

The present invention also relates to a method of treating acute kidney injury including the method for determining renal recovery in individuals according to the present invention and based thereon, treating said individuals without renal replacement therapy or with renal replacement after determination according to the present invention.

**Brief description of the drawings**

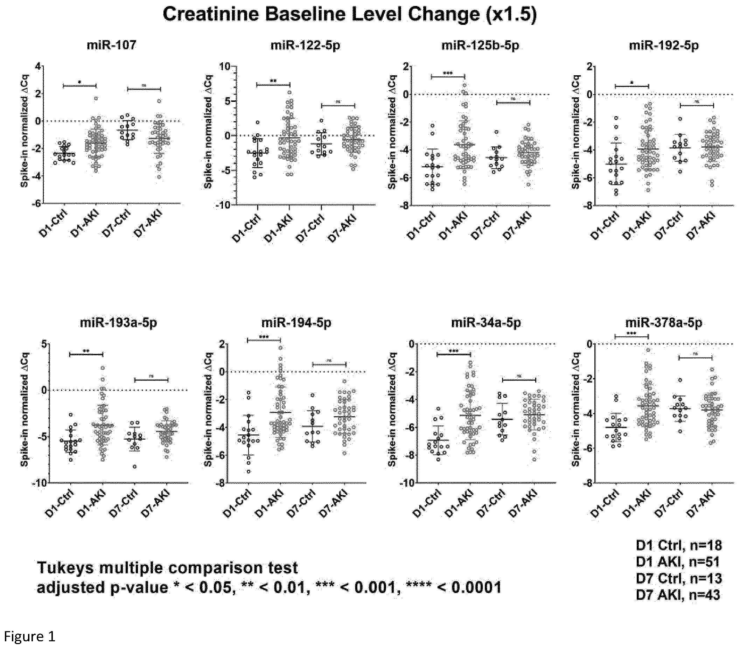

Figure 1: In comparison to patients with recovery of renal function, the 8 depicted miRNAs (i.e. miR-107, miR-122-5p, miR-125-5p, miR-192-5p, miR-193-5p, miR-194-5p, miR-34a-5p and miR-378-5p), measured 24 hrs after AKI (D1-Ctrl) onset, these miRNAs are higher expressed 24 hrs after AKI onset in patients without recovery. D1-Ctrl: miRNA level on day 1, recovery on day 7.
D1-AKI: miRNA level on day 1, no recovery on day 7
D7-Ctrl: miRNA level on day 7, recovery on day 7
D7-AKI: miRNA level on day 1, no recovery on day 7

**Detailed description of the present invention**

In a first aspect, the present invention relates to a method for determining renal recovery in individuals suffering from acute kidney injury (AKI) comprising the steps of

a) providing a sample obtained from said individual suffering from AKI;
b) determining the expression level or amount of at least one of the miRNA selected from the group consisting of miR-192-5p, miR-194-5p, miR-107, miR-122-5p, miR-125b-5p, miR-193a-5p, miR-34a-5p, and miR-378a-3p;
c) comparing the expression level or amount of the at least one miRNA with a reference value of said miRNA;
d) determining whether the individual suffering from acute kidney injury is under renal recovery.

That is, the present inventors recognize that based on the expression level or expression amount of at least one of the miRNA selected from the group consisting of miR-192-5p, miR-194-5p, miR-107, miR-122-5p, miR-125b-5p, miR-193a-5p, miR-34a-5p, and miR-378a-3p, it is possible to identify in individuals suffering from acute kidney injury those individuals with a good prognosis of spontaneous renal recovery.

In this connection, the term "renal recovery" refers to spontaneous decrease in serum creatinine levels within one week after the onset of AKI. That is, based on the expression level or amount of the at least one miRNA, it is possible to prognose or determine whether the individual suffering already from acute kidney injury is already under renal recovery or will undergo renal recovery in the near future.

These individuals do not require renal replacement therapy, in particular, dialysis. There is no risk of adverse effects due to the renal replacement therapy and, in addition, the costs can be kept low and the individual may leave the intensive care unit earlier.

Namely, it has been recognized that in patients suffering from acute kidney injury the expression level or amount of said at least one miRNA is not significantly altered compared to the expression level or amount in individuals not suffering from AKI or compared to a reference value obtained from individuals which suffered from AKI and presently undergo or underwent renal recovery.

In contrast, in individuals suffering from acute kidney injury and requiring renal replacement therapy or being suspected to need renal replacement therapy, the expression level or expression amount are altered namely, are the decreased or increased.

In particular, the expression level or amount of the marker miR-192-5p is increased in case of no recovery. The expression level or amount of the marker miR-194-5p is increased in case of no recovery, miR-107 is increased, miR-122-5p is increased in case of no recovery, miR-125b-5p is increased in case of no recovery, miR-193a-5p is increased in case of no recovery, miR-34a-5p is increased in case of no recovery and miR-378a-3p is increased in case of no recovery.

That is, the level or amount may be determined semi-quantitively or quantitively or relatively to a reference miRNA or reference nucleic acid molecule.

The expression level of the miRNA is based on determining the expression level of the miRNA with suitable methods as detailed below.

In an embodiment of the present invention, the method for determining renal recovery in individuals suffering from acute kidney injury comprise the steps of

a) providing a sample obtained from said individual suffering from AKI;
b) determining the expression level or amount of at least one of the miRNA miR-192-5p and miR-194-5p and of at least one second miRNA whereby the at least second miRNA i) is the other miRNA of miR-192-5p and miR-194-5p and/or ii) is selected from the group consisting of miR-107, miR-122-5p, miR-125b-5p, miR-193a-5p, miR-34a-5p, and miR-378a-3p;
c) comparing the expression level or amount of the at least two miRNA with a reference value of said miRNA;
d) determining whether the individual suffering from acute kidney injury is under renal recovery.

That is, according to this embodiment at least one of the two miRNA miR-192-5p and miR-194-5p is analysed together with at least a second miRNA. The at least two miRNA are analysed and its expression level or amount is determined and compared to the reference value of the respective miRNAs. The second miRNA is either the second of the above two miRNAs, namely, the other miRNA of miR-192-5p and miR-194-5p or, alternatively, is selected from the group consisting of miR-107, miR-122-5p, miR-125b-5p, miR-193a-5p, miR-34a-5p, and miR-378a-3p.

The sequence of the respective miRNA mentioned can be obtained fromMiRBase (www.mirbase.org, Version 22). In particular, the sequences of these miRNAs are as follows:

>hsa-miR-192-5p MIMAT0000222 (SEQ ID No. 1): CUGACCUAUGAAUUGACAGCC;

>hsa-miR-194-5p MIMAT0000460 (SEQ ID No. 2): UGUAACAGCAACUCCAUGUGGA;

>hsa-miR-193a-5p MIMAT0004614 (SEQ ID No. 3): UGGGUCUUUGCGGGCGAGAUGA;

>hsa-miR-107 MIMAT0000104 (SEQ ID No. 4): AGCAGCAUUGUACAGGGCUAUCA;

>hsa-miR-122-5p MIMAT0000421 (SEQ ID No. 5): UGGAGUGUGACAAUGGUGUUUG;

>hsa-miR-125b-5p MIMAT0000423 (SEQ ID No. 6): UCCCUGAGACCCUAACUUGUGA;

>hsa-miR-34a-5p MIMAT0000255 (SEQ ID No. 7): UGGCAGUGUCUUAGCUGGUUGU;

>hsa-miR-378a-5p MIMAT0000731 (SEQ ID No. 8): CUCCUGACUCCAGGUCCUGUGU.

In a further embodiment of the present invention, the combination of the miRNA: miR-192-5p and miR-194-5p, miR-192-5p and miR-193a-5p, or miR-194-5 and miR-34a-5 is determined. As demonstrated, the particular combination of these miRNA allows to determine whether the individuals suffering from acute kidney injury is under renal recovery or will undergo renal recovery with high specificity and sensitivity.

In another embodiment, the expression level or expression amount of at least two, three, four, five, six, or seven of said miRNA, preferably all of the miRNA mentioned above, are determined.

As noted, the determination of the expression level or expression amount can be conducted by known methods. These methods include typically an amplification step of the miRNA. This application step is typically involved in methods and processes including PCR like quantitive PCR reaction (qPCR) as well as next generation sequencing (NGS). A skilled person is well aware and trained in conducting these methods to obtain suitable data allowing the comparison of the obtained data of expression level or expression amount with a reference value. This comparing step may be conducted by applying a data base containing these reference values and may be computed accordingly.

The term "individuals suffering from acute kidney injury" refers to individuals or patients with established acute kidney injury, i.e. based on the creatinine value and/or diruresis, or suspected to suffer from acute kidney injury.

The sample obtained from said individual suffering from AKI may be obtained or collected from sputum, blood including whole blood, plasma, serum, and urine. It is preferred that the sample obtained from said individual is whole blood, plasma or serum. In particular, the sample is a blood plasma sample obtained from said individual. The method according to the present invention can also be applied to samples obtained from individuals being samples derived from a blood sample from said individual. In contrast to prior art methods, the present method based on the particular marker molecules, can be conducted with blood samples which can be obtained at any time from said individuals. In contrast, the prior art methods are based on urine samples only. However, since patients suffering from AKI suffer from oliguria, it is difficult to obtain respective samples from said patients for further analysis.

Surprisingly, the present inventors recognize that the mentioned miRNA molecules present in blood samples allow to determine the likelihood of renal recovery in individuals suffering from acute kidney injury.

In an embodiment the reference value for comparison is obtained from individuals suffering from AKI with renal recovery. In another embodiment, the reference value is obtained from a control group of individuals not suffering from AKI. Further, alternatively, the reference value is obtained from individuals which suffered from AKI in the past and underwent renal recovery. As noted, these values may be obtained from a data base, or, alternatively, these reference value are determine at the same time under the same conditions as it is done for the sample obtained from said individual suffering from AKI to be analyzed.

As noted, the individuals suffering from AKI undergoes renal recovery when the miRNA expression is not significantly altered compared to either a reference value obtained from individuals not suffering from AKI or a reference value obtained from individuals suffered from AKI and underwent renal recovery.

The term "significantly altered" refers a relative or absolute difference to the reference value of at least 10%, like at least 20%, 30%, 40%, 50%, 60%, 70%, 80%, 90%, like at least 100% of increase or decrease.

In an embodiment, the present invention allows to determine renal recovery in said individuals at an early time point. Namely, in an embodiment, the sample is analyzed by determining the expression level or the expression amount of the at least one miRNA as defined herein within 72 hours, preferably 24 hours, like within 12 hours after diagnosis of AKI or after admitted to the intensive care unit due to AKI.

The level or amount of expression of miRNA may be determined by way of microarrays. The skilled person is well aware of suitable methods wherein microarrays are applied.

In another embodiment, the present invention relates to the use of a test kit or kit of parts for determining renal recovery in individuals suffering from AKI. The kit of parts according to the present invention comprises means for determining the expression level or amount of the miRNA as identified herein and instructions of how to use said test kit or kit of parts in a method according to the present invention. Typically, the test kit or kit of parts includes an amplification kit for amplification of the miRNA to be determined. The skilled person is well aware of suitable means for determining the expression level of a miRNA molecule. Said means include components suitable for the amplification thereof as well as means for detecting the amplification products or the expression level of said miRNA generally. For example, microarrays may be parts of the test kit or kit of parts accordingly. Said means may include the suitable probes which may be labeled by suitable labels or marker molecules. Alternatively, the amplification products may be labelled based on using label modified nucleic acid molecules. As noted, the skilled person is well aware of suitable methods for performing amplification labeling accordingly, e. g. using expression microarrays, next generation sequencing etc.

Moreover, the test kit or kit of parts may include components for prior purification or treatment of the sample obtained from said individual to be analyzed. Said compounds or components include means for preparing and providing the miRNA for further use. Said means include enzymes and suitable buffer systems to isolate or purify miRNA while other components are separated.

Further, the present invention provides the *in vitro* use of at least one of the miRNA as defined herein, namely, at least one of the miRNA selected from the group of miR-192-5p, miR-194-5p, miR-107, miR-122-5p, miR-125b-5p, miR-193a-5p, miR-34a-5p, and miR-378a-3p, in determining renal recovery in individuals suffering from AKI while determining the expression level or amount of said miRNA in a sample of an individual suffering from AKI and comparing the expression level or amount with a reference value. In particular, the reference value is a reference value as defined herein.

The method for determining renal recovery in individuals suffering from AKI according to the present invention as well as the use of the test kit or kit of parts or the *in vitro* use as defined herein may be part of a method for treatment of individuals suffering from acute kidney injury. That is, the present invention also relates to a method of treating acute kidney injury in an individual suffering from acute kidney injury including the method according to the present invention of determining renal recovery in individuals. That is, in case of determining that the individual suffering from AKI is under renal recovery or is likely to go under renal recovery, the treatment does not require renal replacement therapy, in particular, dialysis. Rather, the treatment is conservative and includes optimization of volume status, blood glucose levels and avoidance of nephrotoxic drugs.

The present invention will be described further by way of examples, but are limiting the same thereto.

**Examples:**

For initial identification of miRNAs associated with renal recovery, next generation sequencing experiments of human plasma was performed. Differentially expressed miRNAs were further analyzed in a network analysis. Therefore a search in the NCBI Gene database using keywords: "renal Insufficiency" OR "kidney Insufficiency" OR "renal injury" OR "kidney injury" OR "renal failure" OR "kidney failure" were performed. This analysis revealed different numbers of potential miRNA target proteins that were identified via using targetScan 7.2, miRWalk 3 and miRSearch V3.0 (Tab. 1)

Tab.1 Number of relevant protein targets in the context of AKI of the respective miRNAs.

| miRNA | number of target proteins relevant in the context of kidney injury |
|---|---|
| hsa-miR-378a-3p | 30 |
| hsa-miR-193a-5p | 28 |
| hsa-miR-125b-5p | 20 |
| hsa-miR-122-5p | 19 |
| hsa-miR-34a-5p | 18 |
| hsa-miR-107 | 8 |
| hsa-miR-194-5p | 6 |
| hsa-miR-192-5p | 2 |

Finally, the performance of these 8 markers was analyzed using qPCR in a larger cohort of in total 70 patients. Total RNA, including small RNAs, were extracted from 200 μl plasma using the Promega miRNA Plasma/Serum kit according to the instructions of the manufacturer (Promega, US). **Equal volumes were used throughout the workflow.** Total RNA was eluted from beads in 50 μl nuclease-free water. Reverse transcription (RT) was performed in 10 μl reactions with 2 μl total RNA input using the miRCURY RT kit (Qiagen, Germany). RT consisted of polyadenylation and reverse transcription and was performed at 42°C for 60 minutes followed by heat inactivation at 95°C for 5 minutes. The resulting cDNA was diluted 1:20 for qPCR amplification in 10 μl reactions using the miRCURY SYBR® Green mastermix (Qiagen). miRCURY LNA-enhanced dual-specific microRNA primer assays were used (Qiagen). Reactions were performed in 384-well plates. Control assays are synthetic spike-in controls added at the step of RNA extraction (UniSp4), cDNA synthesis (cel-miR-39), and PCR amplification (UniSp3), which allow to determine the yield, homogeneity and overall robustness of RNA extraction, RT, and qPCR amplification. PCRs were performed in a LightCycler 480 II (Roche, Germany): 95°C, 2min activation, followed by 45 cycles of 95°C for 10 seconds and 60°C for 60 seconds. Cq-values were called using the 2nd derivative maximum method available with the Roche software. Melting curve acquisition was performed after 95°C for 10 sec, using continuous acquisition between 55°C and 99°C with a ramp of 0.11 °C/sec and 5 acquisitions per °C.

Normalization of microRNA Cq-values was performed using the UniSp4 RNA spike-in control as internal standard. Delta Cq-values (ΔCq) were calculated by subtracting microRNA Cq-values from RNA spike-in Cq-values according to the formula:

$$\text{delta Cq-value } (\Delta Cq) = Cq(\text{RNA-spike}) - Cq(\text{miRNA}).$$

**Statistical analysis**

Data are presented as medians with interquartile ranges and were analyzed using unpaired Wilcoxon tests with Benjamini-Hochberg correction for multiple testing, respectively. Adjusted p-values < 0.05 were considered statistically significant As shown in fig.1, a set of 8 miRNAs remained in the analysis, all were upregulated in patients without recovery in comparison to patients with recovery.

**Claims**

1. A method for determining renal recovery in individuals suffering from acute kidney injury (AKI) comprising the steps of

   a) providing a sample obtained from said individual suffering from AKI;
   b) determining the expression level or amount of at least one of the miRNA selected from the group consisting of miR-192-5p, miR-194-5p, miR-107, miR-122-5p, miR-125b-5p, miR-193a-5p, miR-34a-5p, and miR-378a-3p;
   c) comparing the expression level or amount of the at least one miRNA with a reference value of said miRNA;
   d) determining whether the individual suffering from acute kidney injury will undergo renal recovery.

2. A method for determining renal recovery in individuals suffering from acute kidney injury (AKI) according to claim 1 comprising the steps of

   a) providing a sample obtained from said individual suffering from AKI;
   b) determining the expression level or amount of at least one of the miRNA miR-192-5p and miR-194-5p and of at least one second miRNA whereby the at least second miRNA i) is the other miRNA of miR-192-5p and miR-194-5p and/or ii) is selected from the group consisting of miR-107, miR-122-5p, miR-125b-5p, miR-193a-5p, miR-34a-5p, and miR-378a-3p;
   c) comparing the expression level or amount of the at least two miRNA with a reference value of said miRNA;
   d) determining whether the individual suffering from acute kidney injury will undergo renal recovery.

3. The method according to claim 1 or 2 wherein at least one of the combination of the miRNA: miR-192-5p and miR-194-5p, miR-192-5p and miR-193-5p, or miR-194-5 and miR-34a-5 is determined.

4. The method according to any one of the preceding claims wherein the expression level or amount of at least two, three, four, five, six, seven of said miRNA, preferably wherein all of said miRNA are determined.

5. The method according to any one of the preceding claims wherein the sample of said individual is collected from sputum, blood including whole blood, plasma, serum and urine preferably obtained from said individual is whole blood, plasma or serum.

6. The method according to claim 4 wherein said sample of the individual is blood plasma.

7. The method according to any one of the preceding claims comprising an amplification step of the miRNA, in particular, by PCR including quantitative PCR reaction (qPCR) and next generation sequencing (NGS).

8. The method according to any one of the preceding claims wherein the reference value is obtained from i) individuals suffering from AKI with renal recovery, or ii) a control group of individuals not suffering from AKI or iii) individuals which suffered from AKI in the past and underwent renal recovery.

9. The method according to any one of the preceding claims wherein the sample is analysed by determining the expression level or amount of the at least one miRNA as defined in claim 1 within 72 hours, preferably 24 hours, like within 12 hours after diagnosis of AKI.

10. The method according to any one of the preceding claims wherein the individual suffering from AKI undergoes renal recovery when the miRNA expression is not significantly altered compared to i) a reference value obtained from individuals not suffering from AKI or ii) a reference value obtained from individuals which suffered from AKI and underwent renal recovery.

11. The method according to any one of the preceding claims wherein the level or amount of expression of miRNA is determined by microarrays.

12. The method according to any one of the preceding claims wherein an increase of the level or amount of expression of the plasma miR-122-5p, -194-5p, -378a-3p, - 125b-5p, -107, -34a-5p, -192-5p, -193a-5p with regard to a reference value of not affected persons or a cohort of individual with established renal recovery is indicative of no renal recovery.

13. The use of a test kit or kit of parts for determining renal recovery in individuals suffering from AKI, said kit or kit of parts comprising means for determining the expression level or amount of the miRNA as identified in claim 1 and instructions of how to use said test kit or kit of parts in a method according to any one of the claims 1 to 12.

14. The use of a test kit or kit of parts according to claim 13 wherein said test kit or kit of parts includes an amplification kit for amplification of nucleic acids.

15. The *in vitro* use of at least one of the miRNA as defined in claim 1 in determining renal recovery in individuals suffering from AKI by determining the expression level or amount of said miRNA in a sample of an individual suffering from AKI and comparing the expression level or amount with a reference value.

Figure 1

Europäisches
Patentamt

European
Patent Office

Office européen
des brevets

**EUROPEAN SEARCH REPORT**

Application Number

EP 20 18 1630

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2012/202201 A1 (THUM THOMAS [DE] ET AL) 9 August 2012 (2012-08-09) * the whole document * * para. 21-24, Fig. 5-6 * | 1-15 | INV. C12Q1/6883 |
| Y | GUO CHUNYUAN ET AL: "Epigenetic regulation in AKI and kidney repair: mechanisms and therapeutic implications", NATURE REVIEWS. NEPHROLOGY, NATURE PUBLISHING GROUP, GB, vol. 15, no. 4, 16 January 2019 (2019-01-16), pages 220-239, XP036733629, ISSN: 1759-5061, DOI: 10.1038/S41581-018-0103-6 [retrieved on 2019-01-16] * p. 234, col. 1 - p. 235, col. 2 * | 1-15 | |
| Y | Edurne M. Fernando Elia M Elisa Laura Ramos García Bermejo Liaño Aguado Fraile Rodríguez Fraile Conde ET AL: "microRNAs in the kidney: novel biomarkers of Acute Kidney Injury", Nefrología: publicación oficial de la Sociedad Española de Nefrología, 1 January 2013 (2013-01-01), pages 826-834, XP055451086, DOI: 10.3265/Nefrologia.pre2013.Aug.12198 Retrieved from the Internet: URL:https://pdfs.semanticscholar.org/ff02/e88141774a7d2d6389135a9e2b80a9ed34e1.pdf * the whole document * * p. 831, col. 1-2 * | 1-15 | TECHNICAL FIELDS SEARCHED (IPC) C12Q |

-/--

The present search report has been drawn up for all claims

| Place of search | Date of completion of the search | Examiner |
|---|---|---|
| Munich | 3 November 2020 | Sauer, Tincuta |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 20 18 1630

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | KIRTI BHATT ET AL: "MicroRNA-34a Is Induced via p53 during Cisplatin Nephrotoxicity and Contributes to Cell Survival", MOLECULAR MEDICINE, vol. 16, no. 9-10, 9 April 2010 (2010-04-09), pages 409-416, XP055746532, WASHINGTON, DC; US ISSN: 1076-1551, DOI: 10.2119/molmed.2010.00002 * the whole document * * p. 414, col. 1 * | 1-15 | |

TECHNICAL FIELDS
SEARCHED      (IPC)

The present search report has been drawn up for all claims

| Place of search | Date of completion of the search | Examiner |
|---|---|---|
| Munich | 3 November 2020 | Sauer, Tincuta |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 20 18 1630

This annex lists the patent family members relating to the patent documents cited in the above-mentioned European search report.
The members are as contained in the European Patent Office EDP file on
The European Patent Office is in no way liable for these particulars which are merely given for the purpose of information.

03-11-2020

| Patent document cited in search report | Publication date | Patent family member(s) | Publication date |
|---|---|---|---|
| US 2012202201 A1 | 09-08-2012 | EP 2484779 A1<br>EP 2505667 A2<br>ES 2513015 T3<br>US 2012202201 A1 | 08-08-2012<br>03-10-2012<br>24-10-2014<br>09-08-2012 |

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2474622 A1

**Non-patent literature cited in the description**

- **GAUDRY. S. et al.** *The New England Journal of Medicine,* 2016, vol. 375, 122-133
- **FAN P-C. et al.** *Human Genomics,* 2016, vol. 10, 29
- **HOSTE E. et al.** *Intensive Care Medicine,* 2020, vol. 46, 943-953